# EUROPEAN PATENT SPECIFICATION

(11) **EP 3 030 319 B1**
(45) Date of publication and mention of the grant of the patent: **04.07.2018**
(21) Application number: 14747668.3
(22) Date of filing: 06.08.2014
(51) Int. Cl.: A61P 19/10, A61P 9/00, A61K 31/222, A61K 31/223, A61K 31/661, A61K 31/663, A61K 31/05, A61K 33/00, A61K 33/06

(54) **COMPOSITIONS COMPRISING VITAMIN K DERIVATIVES AND SALTS**
ZUSAMMENSETZUNGEN MIT VITAMIN-K-DERIVATEN UND SALZEN
COMPOSITIONS CONTENANT DES DÉRIVÉS ET DES SELS DE LA VITAMINE K

(30) Priority: 08.08.2013 GB 201314245
(43) Date of publication of application: 15.06.2016
(73) Proprietor: Kappa Bioscience AS, 0380 Oslo (NO)
(72) Inventor: AUKRUST, Inger Reidun, N-0380 Oslo (NO); MØLLER, Mona, N-0379 Oslo (NO)
(74) Representative: Dehns
(86) International application number: PCT/EP2014/066886
(87) International publication number: WO 2015/018858

(56) References cited:
- WO-A1-2006/080463
- WO-A1-2012/161572
- WO-A1-2013/128037
- DE-A1- 19 964 116
- GB-A- 893 172
- GB-A- 1 424 004
- JP-A- H1 035 256
- TAKATA J ET AL: "Vitamin K prodrugs: 1. Synthesis of amino acid esters of menahydroquinone-4 and enzymatic reconversion to an active form.", PHARMACEUTICAL RESEARCH JAN 1995, vol. 12, no. 1, January 1995 (1995-01), pages 18-23, XP002729809, ISSN: 0724-8741
- TAKATA J ET AL: "Vitamin K prodrugs: 2. water-soluble prodrugs of menahydroquinone-4 for systemic site-specific delivery.", PHARMACEUTICAL RESEARCH DEC 1995, vol. 12, no. 12, December 1995 (1995-12), pages 1973-1979, XP002729810, ISSN: 0724-8741

## Description

This application relates to new formulations of vitamin K1 and K2 provitamins. These formulations can be used as nutraceuticals, e.g. for the fortification of foods or simply in supplements or can be used in pharmaceuticals for the treatment of a variety of conditions known to benefit from the administration of vitamin K1 and K2.

Vitamin K denotes a group of lipophilic and hydrophobic vitamins that are needed for the post-translational modification of certain proteins, mostly required for blood coagulation. Chemically they are 2-methyl-1,4-naphthoquinone derivatives.

Vitamin K is not a single compound, rather it is a series of related homologues. Vitamin K1 is called phylloquinone and has the systematic name all-E-2-methyl-3-(3,7,11,15-tetramethylhexadec-2-enyl)naphthalene-1,4-dione.

Vitamin K2 is a mixture of different molecules based on a naphthoquinone structure and varying lengths of isoprenoid chains. The compound MK-7 (i.e. 7 isoprenyl groups) is depicted below but other components of the vitamin have different numbers of isoprenoid links. Menaquinones have side chains composed of all-E polyprenyl residues; generally they are designated as MK-n, where n specifies the number of isoprenoid repeating units. The minimum value of n is 2.

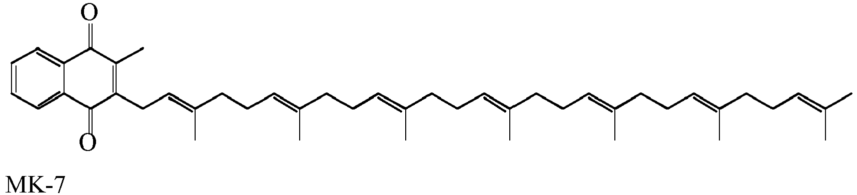

Whilst vitamin K2 occurs naturally in low concentrations in various fermented food products such as cheese and can to a small extent be produced by bacteria in the intestines, its use as a dietary supplement may be beneficial for many populations. Vitamin K2 can be produced by fermentation of soy beans, but it is still an interesting synthetic target as isolation of the vitamin from a natural source is complex and concentrations of the vitamin are low. Moreover, synthesis allows the preparation of particular menaquinones rather than the isolation of a mixture of different menaquinones.

Various individuals have synthesized the menaquinone compounds which form part of vitamin K2 or components thereof. The first synthesis of menaquinones, reported by Isler et al., Helv. Chim Acta 1958, 41, 786-807, used a nonstereospecific approach. Tso and Chen, J Chem Res 1995, 104-105 describes a one pot synthesis of vitamin K although he concentrates on the formation of the naphthoquinone ring as opposed to the side chain of the molecule. His chemistry involves the reaction of 3-substituted isobenzofuranones with vinylic sulphones to form the naphthoquinone ring structure.

Suhara et al, Bioorg Med Chem Lett 17, (2007) 1622-1625, describe various syntheses of menaquinone analogues in which the terminal methyl group is converted to a hydroxyl, aldehyde or acid group.

Naruta, J Org Chem 1980, 45, 4097-4104, describes the synthesis of some vitamin K2 analogues using trialkylallylstannane chemistry to bond the preformed side-chain to the naphthoquinone group.

The present inventors have previously devised a synthetic strategy for the formation of MK-7 and other menaquinones involving the synthesis of a key intermediate in the manufacturing process (WO2010/035000). This process enables the formation of large synthetic quantities of vitamin K2 not previously enabled in the prior art.

The inventors have realised however, that vitamin K1 and especially K2 is not stable towards oxygen and light. Compositions containing vitamin K1 and K2 degrade. Racemisation of the double bonds in the isoprenoid chain leads to inactive vitamin K2 analogues for example, and these double bonds are obviously susceptible to oxidation. Also, the diketone itself is susceptible to oxidation in these vitamins.

The present inventors have also found that vitamins K1 and K2 degrade when formulated in a conventional dosage form such as a tablet in the presence of calcium or magnesium. Moreover, during formulation of the dosage form, there is a still yet further opportunity for degradation. When directly compressed along with some excipients in a tablet such as calcium or magnesium, we observe a serious reduction in the amount of MK-7 present after tabletting, such as up to 30 % reduction in MK-7. As MK-7 is expensive, that is not an acceptable loss on formulation. The MK-7 degradation appears to be accelerated in the presence of calcium or magnesium. As calcium and magnesium are valuable minerals, it is desirable to be able to formulate vitamins in general and MK-7 in particular with calcium and magnesium.

A document by Takata et al. (Pharamceutical Research 1995, vol 12, 18-23) discloses amino acid esters of Menahydroquinone-4, and another by Takata et al. (Pharamceutical Research 1995, vol 12, 1973-1979) discloses water-soluble prodrugs of menahydroquinone-4 for systemic single site delivery. WO2006/080463 discloses therapeutic agents which contain vitamin K hydroquinone esters. GB1424004 discloses di-acetyl esters of vitamin K, and GB893172 discloses phosphoric acid ester derivatives of vitamin K. WO2012/161572 discloses compositions comprising vitamin K2, calcium and magnesium. DE19964116 discloses compositions comprising vitamin K and magnesium.

The inventors have realised that useful provitamins of vitamin K1 and K2 can be prepared from mono or disubstituted derivatives of vitamin K1 and K2, e.g. mono or diester derivatives, where the ketone functionalities of the naphthoquinone ring are protected. The mono or disubstituted vitamin K1 or K2 analogues are capable of undergoing hydrolysis and oxidation in the body to release the equivalent menaquinone type structure. Moreover, the mono or disubstituted compounds are more stable than the vitamin itself in solution and when exposed to light and therefore have a longer shelf life.

The inventors have also found that these vitamin K1 and K2 provitamins can be combined with calcium salts and other metal salts in certain amounts without the degradation issue associated with, for example, MK-7 during processing. Moreover, the provitamin is also much easier to formulate as the loss of active agent during formulation, such as direct compression, is much lower.

The provitamins of the invention are therefore capable of being formulated with calcium and other metal salts with much less degradation than is observed for the combination of vitamin K1 or K2 and calcium. The provitamin does not degrade as rapidly as vitamin K1 or K2 during processing and tabletting (e.g. direct compression) and the provitamin is more stable to light and air meaning it can be used in applications where light and air exposure are common and has a better storage stability such as in beverages containing minerals as well as in tablets.

### Summary of Invention

Thus, viewed from one aspect the invention provides a composition, such as a unit dosage form, comprising:
(A) 0.0001 to 10 wt% of a compound of formula (V): 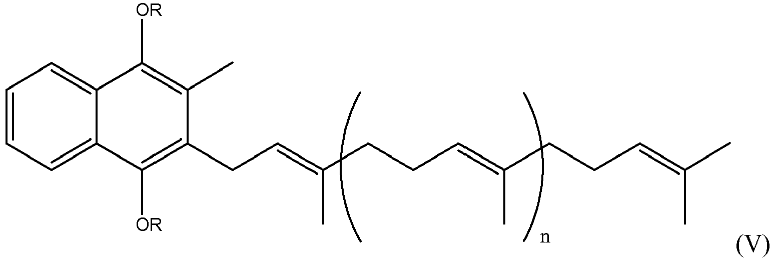 or 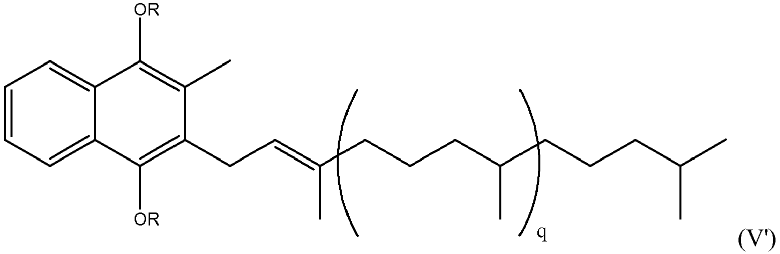
   wherein both R groups are -COCH₂Ar, -COAr, -COC₁₋₆ alkyl group;
   each Ar is an optionally substituted phenyl or naphthyl group, said substituent being a C1-6 alkyl, CHalH₂, CHal₂H, CHal₃, (where Hal is halide), OH, OC₁₋₆-alkyl, COOR⁶;
   each R⁶ is H or C1-6 alkyl;
   q is 2;
   and n is 4 to 7; or a salt or solvate thereof;; and
(B) 10 wt% or more, such as 20 wt% or more of at least one Mg or Ca salt;.

Alternatively viewed, the invention provides a unit dosage form comprising:
(A) 10 to 500 microg of the compound of formula (V) or (V') as hereinbefore defined; and
(B) 10 wt% or more, such as 20 wt% or more of at least one Mg or Ca salt.

Viewed from another aspect the invention provides a nutraceutical or pharmaceutical composition as hereinbefore defined, especially for oral administration.

Viewed from another aspect the invention provides a composition as hereinbefore defined for use in medicine.

Viewed from another aspect the invention provides a composition as hereinbefore defined for use in the treatment of osteoporosis and conditions of the cardiovascular system such as arteriosclerosis or in assisting blood clotting.

Viewed from another aspect the invention provides a process for the formation of a tablet unit dosage form comprising blending
(A) 10 to 500 microg of the compound of formula (V) or (V') as hereinbefore defined; and
(B) 10 wt% or more, such as 20 wt% or more of at least one Mg or Ca salt; to form a blend; and directly compressing that blend to form a tablet.

Viewed from another aspect the invention provides a food or drink fortified with the compound of formula (V) or (V') as hereinbefore defined; and fortified with at least one Mg or Ca salt.

Alternatively viewed, the invention provides a process for the fortification of a food or drink comprising adding to said food or drink a compound of formula (V) or (V') as hereinbefore defined; and
at least one Mg or Ca salt.

### Detailed Description of the Invention

This invention relates to provitamins of vitamin K1 or K2. Analogues of vitamin K1 are of structure: 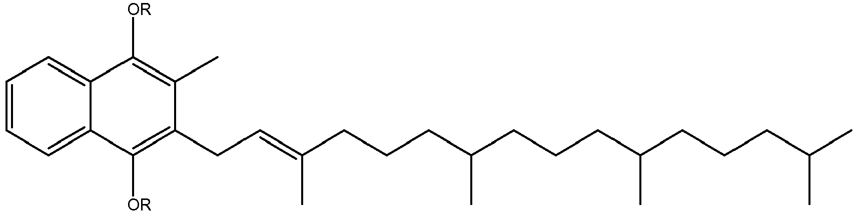 where R is as hereinbefore defined.

Insofar as the compounds are vitamin K2 analogues, the compounds of the invention are preferably analogues of MK-6, MK-7 or MK-8, i.e. n is 4 to 6. MK-9 is also an option, and thus n=7. Most preferably, they are analogues of MK-7 and n is 5. It is thus preferred if the long chain isoprenoid at position 2 on the naphthoquinone ring is

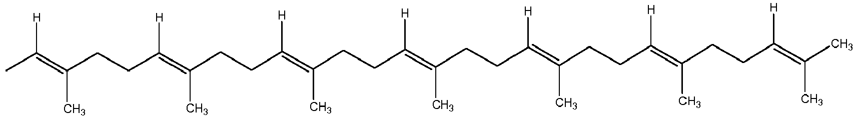

All compounds of the invention are disubstituted analogues of formula (V) or (V'). Thus, both R groups cannot be hydrogen. The compounds of the invention are disubstituted. In one embodiment of the invention both R groups are acetate (thus forming -OCOCH₃ at the 1 and 4 position). It is however, also within the scope of the invention for both R groups not to be acetate.

It is within the scope of the invention for the substituent groups R used in a compounds of formula (V) or (V') to be the same or different however, it is preferred if these are the same. Bis substituted compounds are generally more stable. Preferred ester groups are methyl esters, ethyl esters or phenyl esters.

A further option is compounds in which R is -CO(CH₂)ₚAr, such as benzyl. Ar is preferably Ph or 4-CF₃-Ph-.

Where the compounds of the invention comprise an alkyl chain, e.g. as part of the ester, this alkyl chain may contain a substituent selected from -OR², N(R²)₂, or COOR². This substituent therefore provides polarity to the molecule and aids its dissolution in the body. If present, preferably one such group should be present. Preferably, that group should be OH. Preferably no such substituent is present.

It is preferred if the compound of the invention is of formula (V).

In a further preferred embodiment however, the compounds of the invention comprise at least one ester OCO- at the OR position.

Preferred compounds of the invention are of formula (II) or (IV): 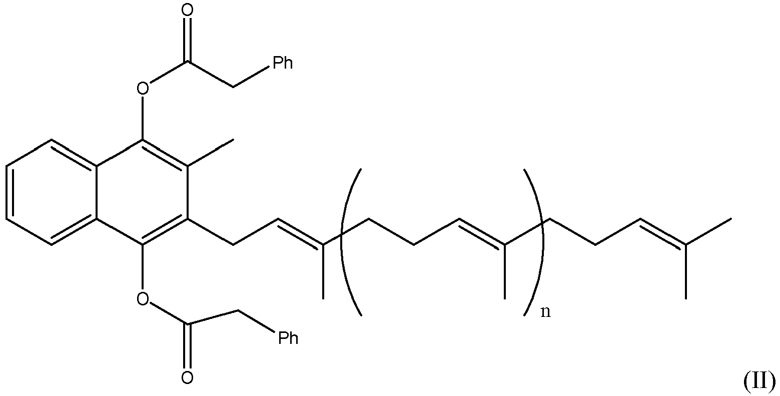 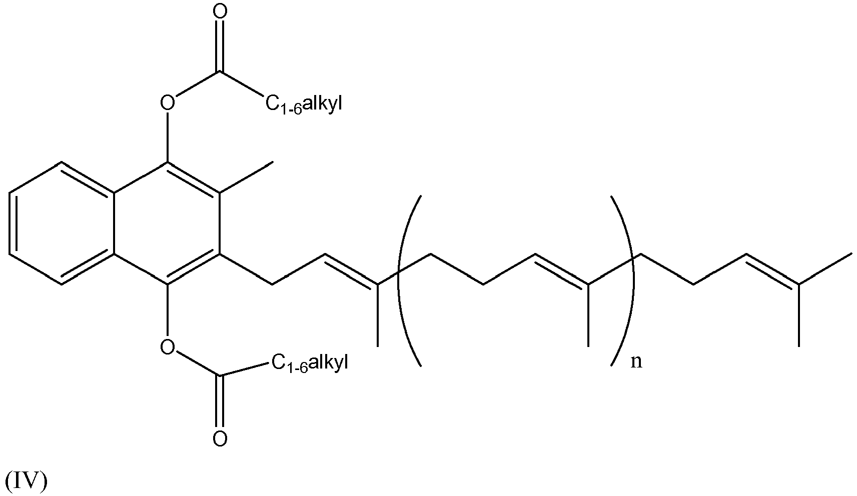 where n is 5;
Further preferred compounds are those of formula (V) 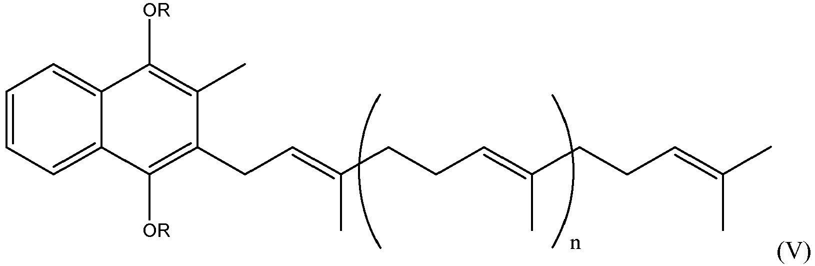 or 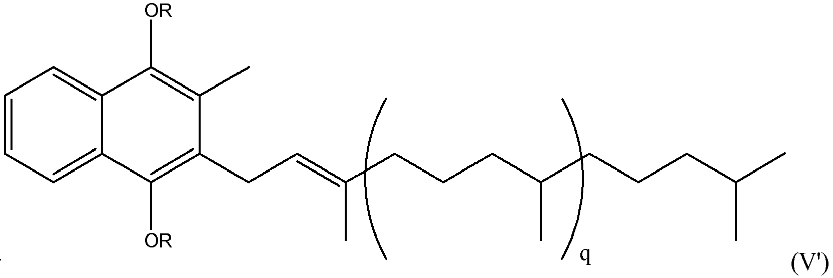
wherein both R groups are COCH₂Ar, COAr or -COC₁₋₆ alkyl group;
each Ar is an optionally substituted phenyl or naphthyl group, said substitutent being a C1-6 alkyl, CHalH₂, CHal₂H, CHal₃, OH, OC1-6-alkyl, COOR⁶;
R⁶ is H or C1-6 alkyl;
q is 2;
and n is 4 to 7; or a salt or solvate thereof.

It has surprisingly been found that the compounds of the invention have a much longer shelf life than their corresponding diketone vitamin K1 or K2 analogue. Without wishing to be limited by theory, it is envisaged that the claimed compounds are less susceptible to oxidation.

It is important however, that the OR group is capable of hydrolysis and oxidation within the body to yield the native MK-n analogue and hence vitamin K2 type structure. The claimed structures are all based on readily hydrolysable ester type linkages.

The compounds (II) and (IV) above, and in particular the compound 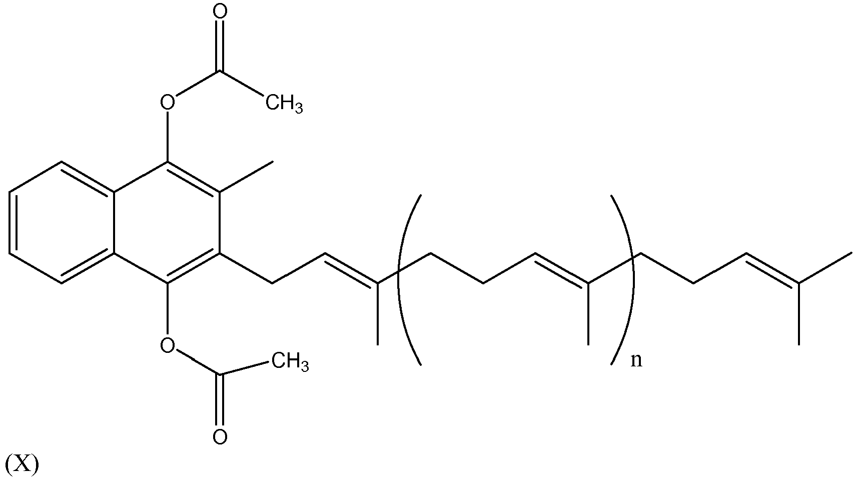 where n is where n is 4 to 7, preferably 4 to 6; especially 5 are interesting.

It is envisaged that these compounds are of particular utility in fortifying dietary supplements, food and beverages such as soft drinks. Thus the invention further provides a dietary supplement fortified with 15 to 400 microg of a compound of formula (Z) 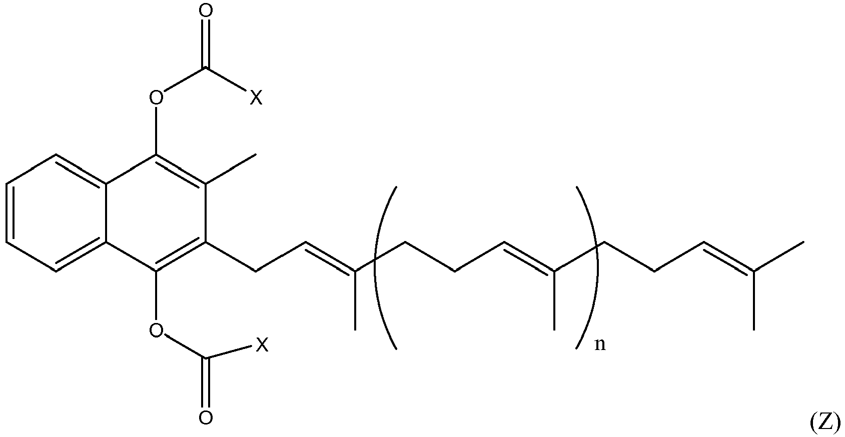 or 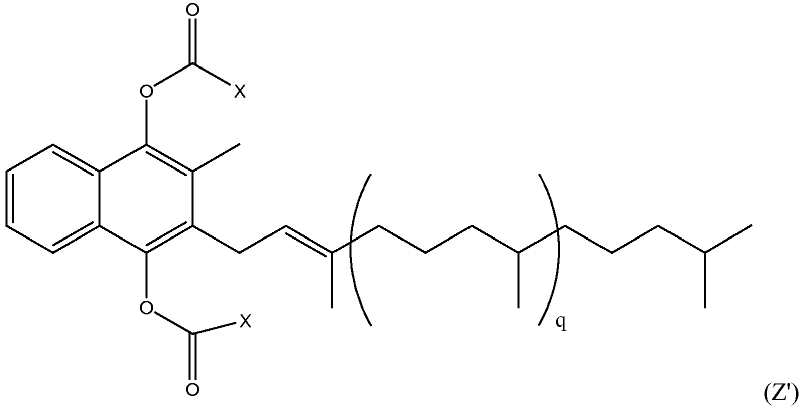 where X is methyl, ethyl or CH₂Ph, q is 2 and n is where n is 4 to 7, preferably 4 to 6; especially 5.

Viewed from another aspect the invention provides food or beverage fortified with a compound of formula (Z) 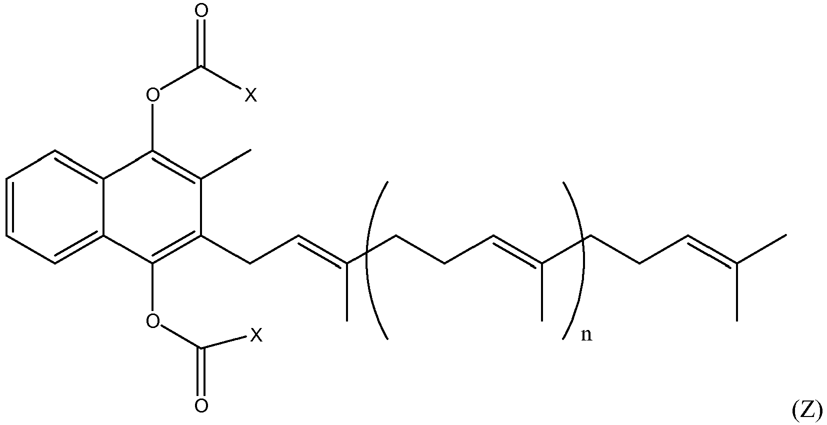 or 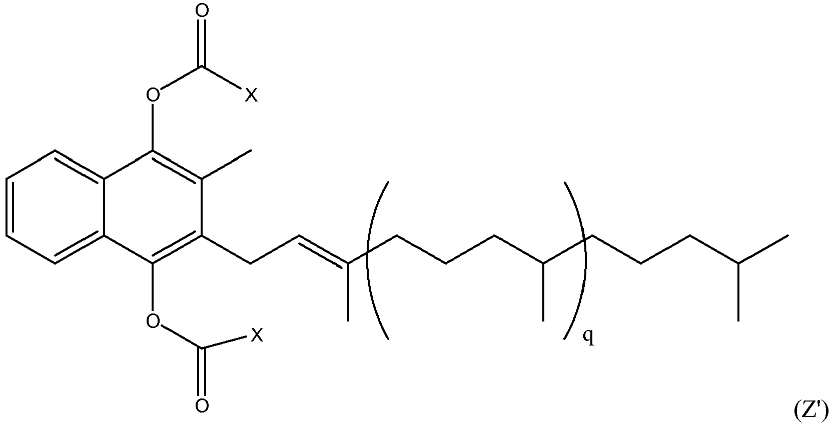 where X is methyl, ethyl or CH₂Ph, q is 2 and n is where n is 4 to 7, preferably 4 to 6; especially 5. There may be 15 to 400 microg of compound in the food or beverage.

It is also preferred if the dietary supplement, food or beverage is also fortified with at least one metal salt as hereindefined.

### Synthesis

This section discusses the synthesis of vitamin K2 analogues. Vitamin K1 analogues can be made using the same chemistry starting from phylloquinone. The compounds of the invention can be synthesized from the corresponding menaquinone compound, e.g. MK-7. Menaquinone compounds of use as starting materials can be prepared following the protocols of WO2010/035000 which is herein incorporated by reference. Naturally occurring vitamin K2 could also be used here. It will be appreciated therefore that the starting menaquinone reactant might contain a mixture of different MK-n compounds (where n is the chain length). Naturally occurring vitamin K2 is formed from chains of differing lengths.

The invention therefore covers a composition in which there are a mixture of compounds of formula (V) as hereinbefore defined in which the value of n varies, e.g. a mixture comprising MK-6, MK-7 and MK-8 analogues of formula (V).

The incorporation of an ester group on the ketone functionality of the ring can be achieved by treatment in the presence of, for example, an anhydride and zinc such as AC₂O/Zn. The presence of a base such as sodium acetate also helps the synthesis. Other anhydrides of use include, *inter alia,* propionic anhydride and so on. The general protocol is summarised in scheme 1

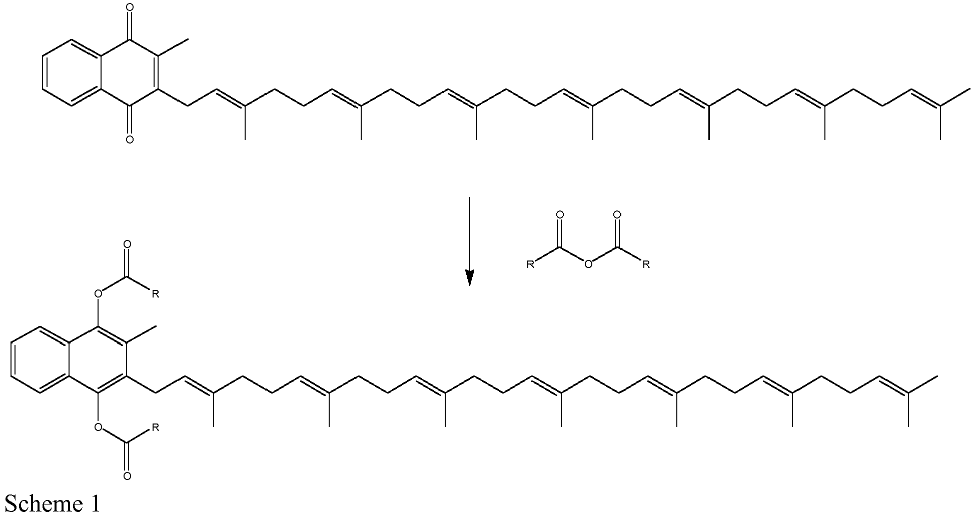

It will be appreciated that the OR groups might be added before the final molecule synthesis is completed. In particular, the present inventors have previously taught a process for the manufacture of vitamin K2 relying on Kumada or Suzuki chemistry to couple isoprenoid chains to naphthoquinone rings. That chemistry could be employed here.

It is preferred in WO2010/035000, if the 7 unit isoprenoid chain of MK-7 is developed by coupling a pentraprenol to a naphthoquinone carrying 2-isoprenoid units. The key intermediate in this process can be provided with the OR groups of this invention before being coupled to the pentaprenol. The key intermediate in the synthesis is therefore of formula (VII): 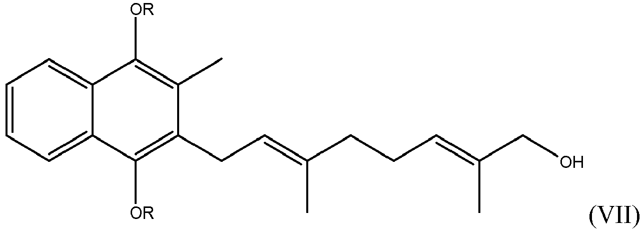 wherein R is as hereinbefore defined. In order to couple this compound to a pentaprenol type structure, it is useful to convert the hydroxyl group to a better leaving group, especially a halo group. A further aspect of the invention therefore relates to the compound of formula (VI): 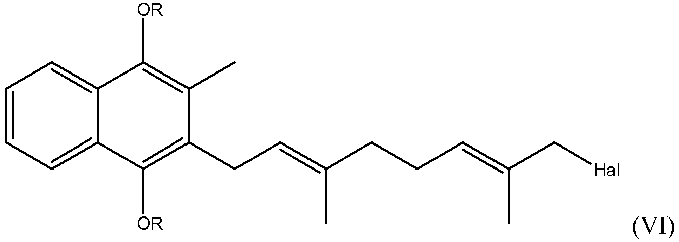 wherein Hal is a halide, especially bromide and R is as hereinbefore defined.

The skilled person will be able to devise various procedures for introducing the necessary R groups onto the compounds of formula (V). For example, the skilled person could follow the ideas in scheme 3:

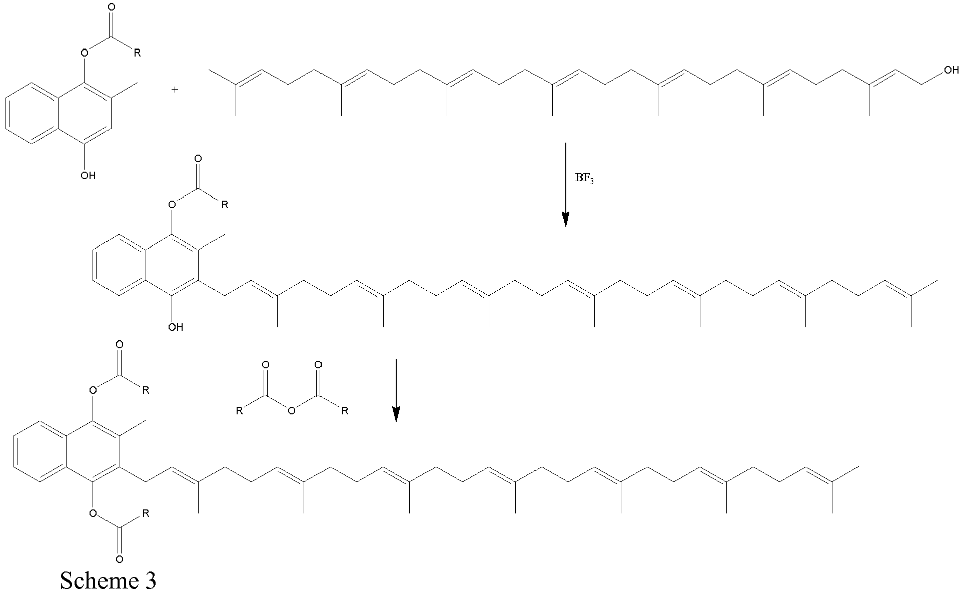

In this scheme, a monosubstituted naphthoquinone is coupled with the seven member isoprenoid chain and then a further carboxyl group is coupled to the free hydroxyl. It will be appreciated therefore that there are many options available to the skilled person here.

The compounds of formula (V) may also be present as salts. Salts of the compounds of formula (V) are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable salts are defined to comprise the therapeutically active non-toxic acid addition salt forms that the compounds according to formula (V) are able to form. Said salts can be obtained by treating the base form of the compounds according to formula (V) with appropriate acids, for example inorganic acids, for example hydrohalic acid, in particular hydrochloric acid, hydrobromic acid, sulphuric acid, nitric acid and phosphoric acid ; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid.

Conversely said acid salt forms can be converted into the free base form by treatment with an appropriate base.

The compounds according to formula (V) containing acidic protons may also be converted into their therapeutically active non-toxic base salt forms by treatment with appropriate organic and inorganic bases. Appropriate base salt forms comprise, for example, the ammonium salts, the alkaline and earth alkaline metal salts, in particular lithium, sodium, potassium, magnesium and calcium salts, salts with organic bases, e.g. the benzathine, N-methyl-D-glucamine, hybramine salts, and salts with amino acids, for example arginine and lysine.

Conversely, said base salt forms can be converted into the free acid forms by treatment with an appropriate acid.

The pharmaceutically acceptable acid addition salt forms of the compounds of formula (V) are the preferred pharmaceutically acceptable salt forms of the compounds of formula (V).

The invention also encompasses solvates of the compounds of formula (V). The term solvate comprises the solvent addition forms of the base compound as well as the pharmaceutically acceptable salts thereof, which the compounds of formula (V) are able to form. Examples of such solvent addition forms are e.g. hydrates, alcoholates and the like.

It will of course be possible to use a mixture of compounds of formula (V) in the compositions of the invention.

Exactly the same synthetic principles apply to compounds of formula (V') herein.

### Compositions

Compositions of the invention may comprise 0.0001 to 10 wt% of the compound of formula (V) or (V'), such as 0.001 to 1 wt% of the compound of formula (V) or (V') or 0.01 to 1 wt%. Typically, there is 10 to 500 microg of the compound of formula (V) or (V') such as 25 to 200 microg in the composition of the invention, such as a single dosage form, e.g. a tablet or capsule. It is most preferred therefore if the composition of the invention is a tablet and comprises 25 to 200 microg of the compound of formula (V) or (V').

If the composition of the invention is being used to fortify food or drink, then the amount added will reflect the food in question and will be designed to offer the consumer between 10 to 500 microg of the compound of formula (V) or (V') in a typical serving of the food in question. Thus, the actual concentration of the compounds of formula (V) or (V') might be higher in a food like butter where amounts consumed are small, compared to milk where much more is consumed.

Alternatively viewed, the amount of the compound of formula (V) or (V') added is designed to deliver 10 to 500 microg of the compound of formula (V) or (V') to a consumer in a day.

The compound of formula (V) or (V') is combined with at least one Mg or Ca salt. Preferred ions are based on a 2+ ion. Preferably the salt is a calcium or magnesium salt. A mixture of salts may also be used.

The salt can preferably be any pharmaceutically acceptable salt such as a halide, nitrate, stearate, sulphate, carbonate, glycerophosphate, hydrogencarbonate, dihydro- or anhydro- phosphate.

In a most preferred embodiment, the salt is a calcium salt, ideally calcium carbonate.

We have observed that when MK-7 is formulated with calcium salts, it degrades rapidly. The use of a provitamin deals with this problem. We are therefore able to formulate MK-7 provitamins with calcium salts without the serious levels of degradation observed with MK-7.

The amount of salt in the composition can be at least 10 wt%, such as at least 20 wt% or at least 30 wt%. Where there a blend of salts is present, it is still required that one of these is present in an amount of at least 10 wt% of the composition. Whilst these values are appropriate for dietary supplements, it will be appreciated that the addition of salts to food or drink will typically be at lower concentrations.

A typical unit dosage form may be 700 to 1000 mg in weight. Ideally 40 wt% or more of the dosage form comprises the salt, ideally the calcium or magnesium salt, such as 60 wt% or more, ideally 75 wt% or more.

In a most preferred embodiment the composition of the invention contains both Ca and Mg salts with an excess of the Ca salt.

As well as the salt and the provitamin, other known excipients may be used.

A further preferred option involves the addition of other vitamins or provitamins and/or other mineral salts such as Zn and Se along with calcium.

The compounds of the invention may also be used in combination therapy with other active agents.

It will be appreciated that pharmaceutical compositions for use in accordance with the present invention may be in any suitable form but ideally, they are for oral administration, ideally in the form of tablets. Such tablets may be formulated in conventional manner.

The composition of the invention may also be used to fortify foods, or just be used as a nutraceutical, i.e. as a dietary supplement such as a vitamin pill or multivitamin pill. The use therefore of the provitamins of the invention in combination with other vitamins or provitamins is especially preferred.

We have shown that our compounds provide a long lasting effect within the body. In our rat model, we have shown that after 12 hrs, the compounds of invention are able to provide the same level of active component as MK-7 itself. This makes the compounds attractive for example for once a day administration.

The compounds of the invention can be administered for immediate-, delayed-, modified-, sustained-, pulsed-or controlled-release applications.

Examples of pharmaceutically acceptable disintegrants for oral compositions useful in the present invention include, but are not limited to, starch, pre-gelatinized starch, sodium starch glycolate, sodium carboxymethylcellulose, croscarmellose sodium, microcrystalline cellulose, alginates, resins, surfactants, effervescent compositions, aqueous aluminium silicates and crosslinked polyvinylpyrrolidone.

Examples of pharmaceutically acceptable binders for oral compositions useful herein include, but are not limited to, acacia; cellulose derivatives, such as methylcellulose, carboxymethylcellulose, hydroxypropylmethylcellulose, hydroxypropylcellulose or hydroxyethylcellulose; gelatin, glucose, dextrose, xylitol, polymethacrylates, polyvinylpyrrolidone, sorbitol, starch, pre-gelatinized starch, tragacanth, xanthane resin, alginates, magnesium-aluminum silicate, polyethylene glycol, acacia gum or bentonite.

Examples of pharmaceutically acceptable fillers for oral compositions (other than the calcium salts required herein) include, but are not limited to, lactose, anhydrolactose, lactose monohydrate, sucrose, dextrose, mannitol, sorbitol, starch, cellulose (particularly microcrystalline cellulose).

Examples of pharmaceutically acceptable lubricants useful in the compositions of the invention include, but are not limited to, talc, polyethylene glycol, polymers of ethylene oxide, sodium lauryl sulfate, sodium oleate, sodium stearyl fumarate, and colloidal silicon dioxide.

Examples of suitable pharmaceutically acceptable odorants for the oral compositions include, but are not limited to, synthetic aromas and natural aromatic oils such as extracts of oils, flowers, fruits (e.g., banana, apple, sour cherry, peach) and combinations thereof, and similar aromas. Their use depends on many factors, the most important being the organoleptic acceptability for the population that will be taking the pharmaceutical compositions.

Examples of suitable pharmaceutically acceptable dyes for the oral compositions include, but are not limited to, synthetic and natural dyes such as titanium dioxide, beta-carotene and extracts of grapefruit peel.

Suitable examples of pharmaceutically acceptable sweeteners for the oral compositions include, but are not limited to, aspartame, saccharin, saccharin sodium, sodium cyclamate, xylitol, mannitol, sorbitol, lactose and sucrose. Suitable examples of pharmaceutically acceptable buffers include, but are not limited to, citric acid, sodium citrate, sodium bicarbonate and dibasic sodium phosphate.

Suitable examples of pharmaceutically acceptable surfactants include, but are not limited to, sodium lauryl sulfate and polysorbates.

Suitable examples of pharmaceutically acceptable preservatives include, but are not limited to, various antibacterial and antifungal agents such as solvents, for example ethanol, propylene glycol, benzyl alcohol, chlorobutanol, quaternary ammonium salts, and parabens (such as methyl paraben, ethyl paraben, propyl paraben, etc.).

Suitable examples of pharmaceutically acceptable stabilizers and antioxidants include, but are not limited to, ethylenediaminetetriacetic acid (EDTA), thiourea, tocopherol and butyl hydroxyanisole.

### Applications

Vitamin K2 and hence MK-7 has well documented therapeutic applications and the provitamins of vitamin K2 produced in this invention are suitable for all known therapeutic applications of vitamin K2. It can also be used as a food supplement or in any nutraceutical product, e.g. as a vitamin supplement.

Conditions in which vitamin K2 administration may assist treatment include osteoporosis and bone related disorders, cardiovascular health in general such as arteriosclerosis, myocardial infarction, calcification of blood vessels, diabetes, male infertility, conditions associated with inflammation and so on.

The compounds of the present invention may be utilized alone or in combination with one or more other drugs in the treatment, prevention, control, amelioration, or reduction of risk of diseases or conditions for which compounds of Formula (V) may have utility.

The compounds of formula (V) have utility in, *inter alia,* the treatment of osteoporosis, cancer, diabetes, male infertility or cardio-vascular disease. The compounds may also be used as vitamin supplements or in any other known application of vitamin K2, e.g. for injection into new-born infants to aid blood clotting.

Vitamin K1 is well known to have importance in the blood clotting cascade and is required for bone protein formation.

The compounds of the invention may be taken once a day, twice a day, more often or less often depending on the purpose of administration, preferably once a day. It is particularly preferred that analogues of MK-7 can be administered once a day whereas analogues of other menaquinones such as MK-4 cannot.

The dose and the administration frequency will also depend on the use in question, e.g. whether for clinical use or via a supplement. A dosage of 20 to 250 micro g/day is suitable as a food supplement. A dosage of 120 - 500 micro g/day may suitable as a pharmaceutical product.

In particular the compositions of the invention can be used in food fortification, e.g. of natto.

A further major advantage of the presence compounds is that they may be taken at any time. Conventional vitamin K supplements are taken with meals as consuming them along with fat enhances the bioavailability of the vitamin K in the body. Many consumers, however, fail to remember to take the product with a meal or perhaps eat the vitamin K supplement with a meal such as breakfast which often has almost no fat in it. The bioabsorption of the vitamin K supplement is therefore reduced in these circumstances.

The compound of the present invention are less dependent on the presence of fat and offer the ability to be taken at any time or with breakfast as there is no requirement to administer the compounds with a fatty additive.

The invention will now be further described with reference to the following non limiting examples.

In the examples which follow:

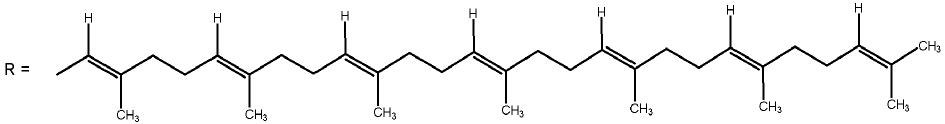

### Example 1

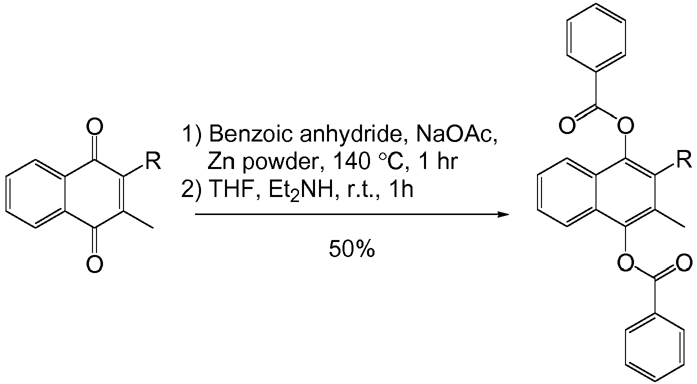

2-((2*E,*6*E,*10*E,*14*E,*18*E,*22*E*)*-*3*,*7*,*11*,*15*,*19*,*23*,*27*-*Heptamethyloctacosa-2,6,10,14,18,22,26-heptaen-1-yl)-3-methylnaphthalene-1,4-dione (1.00 g, 1.34 mmol), benzoic anhydride (6.00 g, 26.52 mmol), NaOAc (0.134 g, 1.64 mmol) and Zn powder (0.31 g, 4.74 mmol) were added together and heated to 140 °C. After 1 h at 140 °C the reaction mixture was cooled down to r.t. and diluted with THF (40 mL). Et₂NH (20 mL) was added and the reaction mixture was stirred for another hour after which heptane (50 mL) was added. The resulting mixture was filtrated and the solvent was removed under reduced pressure. The crude product was purified by flash chromatography (heptane : EtOAc gradient) to obtain 0.58 (50%) of 2-((2*E*,6*E*,10*E,*14*E,*18*E*,22*E*)-3,7,11,15,19,23,27-heptamethyl-octacosa-2,6,10,14,18,22,26-heptaen-1-yl)-3-methylnaphthalene-1,4-diyl dibenzoate as a dark yellow oil.
¹H NMR (400 MHz, CDCl₃) δ 8.34 (t, *J* = 7.8, 4 H), 7.80 - 7.65 (m, 4 H), 7.62 - 7.52 (m, 4 H), 7.44 - 7.36 (m, 2 H), 5.18 - 5.00 (m, 7 H), 3.60 - 3.38 (m, 2 H), 2.31 (s, 3 H), 2.12 - 1.85 (m, 23 H), 1.66 (s, 3 H), 1.63 - 1.47 (m, 22 H).
¹³C NMR (101 MHz, CDCl₃) δ 166.51, 143.06, 142.86, 136.49, 135.36, 135.15, 135.12, 134.04, 134.02, 133.95, 131.46, 130.90, 130.64, 129.42, 129.39, 129.02, 128.95, 127.49, 126.76, 126.63, 126.53, 124.64, 124.64, 124.46, 124.25, 121.78, 121.57, 121.40, 39.97, 39.95, 39.84, 27.38, 27.38, 27.00, 26.96, 26.94, 26.91, 26.79, 25.91, 25.91, 17.90, 16.57, 16.57, 16.26, 16.24, 16.22, 13.45.
MS: *m*/*z* [M + Na]⁺calcd for C₆₀H₇₄NO₄: 881.5485; found: 881.4

### Example 2 deleted

### Example 3 deleted

### Example 4 deleted

### Example 5 deleted

### Example 6 deleted

### Example 7

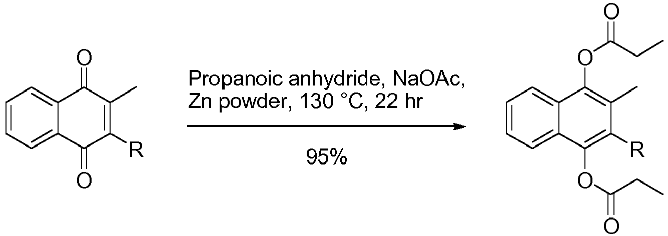

2-((2*E*,6*E*,10*E*,14*E*,18*E*,22*E*)-3,7,11,15,19,23,27-heptamethyloctacosa-2,6,10,14,18,22,26-heptaen-1-yl)-3-methylnaphthalene-1,4-dione (324 mg, 0.50 mmol), propanoic anhydride (7.50 mL, 80 mmol), NaOAc (50 mg, 0.60 mmol) and Zn powder (100 mg, 1.55 mmol) were added together and heated to 130 °C. The reaction mixture was stirred for 30 minutes. After cooling to room temperature, the reaction mixture was poured into water and extracted with CHCl₃ (x2) and the combined organic phases were dried (Na₂SO₄), filtrated and the solvent was removed under reduced pressure. The crude product was purified by flash chromatography (heptane : EtOAc gradient) to give 250 mg (66%) of 2-((2*E,*6*E,*10*E,*14*E,*18*E*,22*E*)-3,7,11,15,19,23,27-heptamethyloctacosa-2,6,10,14,18,22,26-heptaen-1-yl)-3-methylnaphthalene-1,4-diyl dipropionate as a yellow oil.
¹H NMR (300 MHz, CDCl3) δ 7.76 - 7.55 (m, 2H), 7.55 - 7.33 (m, 2H), 5.09 (dt, *J* = 5.4, 3.7, 7H), 3.39 (d, *J* = 4.7, 2H), 2.77 (qd, *J* = 7.6, 5.3, 4H), 2.22 (s, 3H), 2.15 - 1.82 (m, 24H), 1.76 (s, 3H), 1.67 (s, 3H), 1.58 (d, *J* = 5.9, 18H), 1.37 (td, *J* = 7.6, 6.1, 6H).
¹³C NMR (75 MHz, CDCl3) δ 173.06, 172.64, 136.40, 135.38, 135.11, 131.45, 126.44, 126.35, 124.60, 124.44, 124.13, 121.56, 121.38, 77.65, 77.23, 76.81, 39.94, 39.80, 27.74, 27.21, 27.02 - 26.89, 26.80, 25.92, 17.90, 16.59, 16.24, 13.26, 9.63.
MS: *m*/*z* [M + Na]⁺calcd for C₅₂H₇₄O₄:785.55; found: 785.7

### Example 8 deleted

### Example 9 deleted

### Example 10 deleted

### Example 11 deleted

### Example 12 deleted

### Example 13

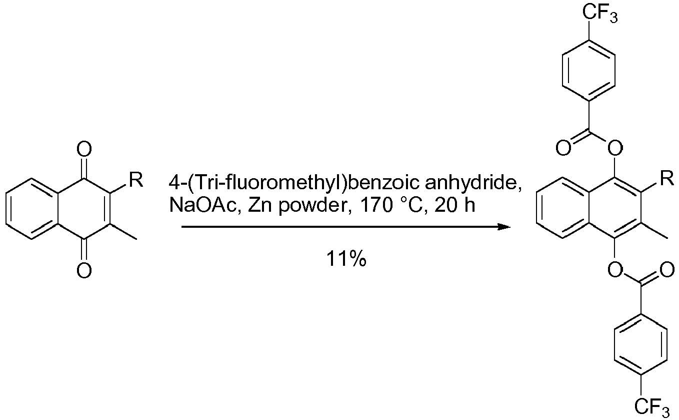

2-((2*E*,6*E*,10*E*,14*E*,18*E*,22*E*)-3,7,11,15,19,23,27-Heptamethyloctacosa-2,6,10,14,18,22,26-heptaen-1-yl)-3-methylnaphthalene-1,4-dione (0.524 g, 0.807 mmol), 4-(trifluoromethyl)benzoic anhydride (0.757 g, 2.09 mmol), NaOAc (87.3 mg, 1.06 mmol) and Zn powder (0.156 g, 2.38 mmol) were added together and heated to 170 °C. After 23 h at 170 °C the reaction mixture was cooled down to r.t. and diluted with THF (40 mL). Et₂NH (20 mL) was added and the reaction mixture was stirred for another hour after which heptane (50 mL) was added. The resulting mixture was filtrated and the solvent of the filtrate was removed under reduced pressure. The crude product was purified by HPLC to obtain 83.7 mg (11%) of 2-((2*E,*6*E,*10*E,*14*E,*18*E*,22*E*)-3,7,11,15,19,23,27-heptamethyloctacosa-2,6,10,14,18,22,26-heptaen-1-yl)-3-methylnaphthalene-1,4-diyl bis(4-(trifluoromethyl)benzoate).
¹H NMR (400 MHz, CDCl₃) δ 8.45 (t, *J* = 8.6, 4 H), 7.88 - 7.81 (m, 4 H), 7.76 - 7.65 (m, 2 H), 7.45 - 7.40 (m, 2 H), 5.17 - 4.98 (m, 7 H), 3.59 - 3.34 (m, 2 H), 2.31 (s, 3 H), 2.15 - 1.83 (m, 24 H), 1.65 (s, 3 H), 1.62 - 1.46 (m, 18 H), 1.24 (s, 3 H).
MS: *m*/*z* [M + Na]⁺calcd for C₆₂H₇₂F₆O₄: 1017.52; found: 1017.3

### Example 14 deleted

### Example 15

### 2-((2E,6E,10E,14E,18E,22E)-3,7,11,15,19,23,27-heptamethyloctacosa-2,6,10,14,18,22,26-heptaen-1-yl)-3-methylnaphtalene-1,4-diyl dipropionate

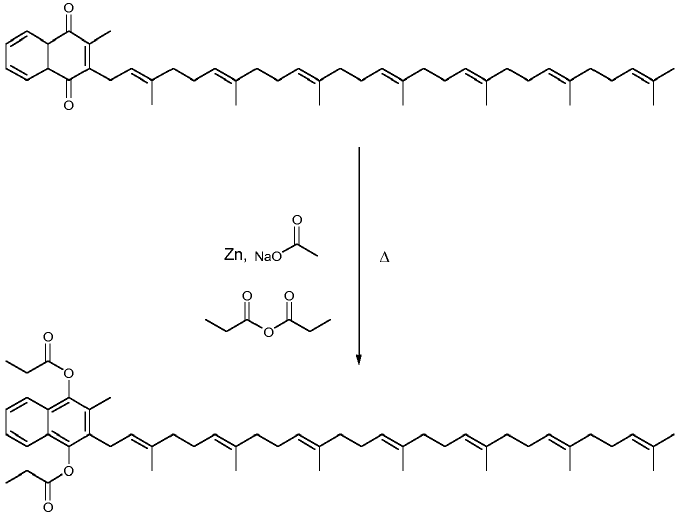

A mixture of Vitamin K2 MK-7 (MK:3:94, 324 mg, 0.5 mmol), zinc dust (100 mg, 1.55 mmol), anhydrous sodium acetate (50 mg, 0.60 mmol) and propionic anhydride (7.5 ml, 80 mmol) was heated to 130 °C during 30 min., after cooling to room temperature poured into water (100 ml) and extracted with CHCl₃ (2 x 50 ml). The combined organic phase was dried (Na₂SO₄), filtered and concentrated. Excess propionic anhydride was distilled off under reduced pressure and the remaining oil purified by flash chromatography (heptane : EtOAc 95:5) to afford 250 mg yield of the title compound as a colorless solid.
¹H NMR (300 MHz, CDCl₃): δ 7.73-7.66 (2H, m), 7.49-7.46 (2H, m), 5.14-5.10 (7H, m), 3.44, 3.42, 2.83-2.77 (m, 4H), 2.26 (s, 3H), 2.24-1.95 (m, 24H), 1.80 (s, 3H), 1.71 (s, 3H), 1.63-1.60 (m, 18H), 1.43-1.40 (m, 6H).
¹³C NMR (75 MHz, CDCl₃): δ 173.4, 172.6, 142.7, 136.60, 135.3, 131.7, 130.7, 127.3, 126.6, 124.7, 124.3, 121.6, 40.1, 27.9, 27.4, 27.1, 26.0, 18.0, 16.7, 16.4, 13.6, 9.9

### Example 16:

### Acetic acid 3-(3,7,11,15,19,23,27-heptamethyl-octacosa-2,6,10,14,18,22,26-heptaenyl)-4-hydroxy-2-methyl-naphthalen-1-yl ester

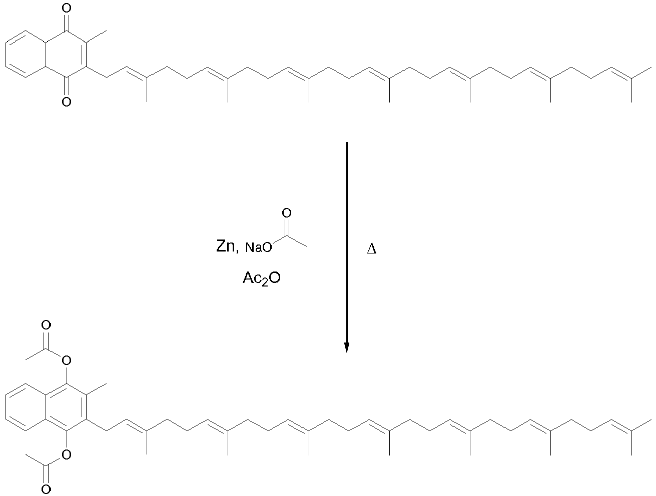

### (Light was off in the hood during reaction and work-up.)

A mixture of VitaminK2 MK-7 (0.1997 g, 0.31 mmol), Zn (0.064 g, 0.98 mmol) and sodium acetate (0.0304 g, 0.37 mmol) in acetic acid anhydride (4.7 ml) was refluxed under N₂-atmosphere for 30 minutes. The reaction mixture was cooled to room temperature, diluted with CH₂Cl₂ (50 ml), filtered, washed with water (20 ml) and brine (20 ml), dried (Na₂SO₄), and evaporated under reduced pressure to yield 0.160 g (71%) of the crude title compound as a colorless solid.
¹H NMR (300 MHz, CDCl₃) δ 7.60 - 7.78 (m, 2H), 7.38 - 7.53 (m, 2H), 4.90 - 5.24 (m, 7H), 3.43 (s, 2H), 2.49 (s, 3H), 2.47 (s, 3H), 2.25 (s, 3H), 1.85 - 2.16 (m, 24H), 1.79 (s, 3H), 1.69 (s, 3H), 1.59 (d, *J* = 5.6 Hz, 18H).
¹³C NMR (75 MHz, CDCl₃) δ 169.58, 169.15, 142.71, 142.42, 136.44, 135.34, 135.07, 135.06, 135.04, 135.02, 131.39, 130.46, 127.08, 126.48, 126.40, 126.38, 126.28, 124.53, 124.39, 124.34, 124.05, 121.52, 121.32, 121.22, 39.87, 39.75, 27.19, 26.90, 26.84, 26.81, 26.71, 25.85, 20.84, 20.77, 17.83, 16.53, 16.19, 16.16, 13.21. MS (electrospray) (pos): 757/758/759 (M+Na)⁺

### Stability

The light stability of MK-7 was compared to compounds of the invention as hereinbefore described. Compounds were dissolved separately in ethyl acetate or MCT oil, transferred to glass vial (clear glass) and placed on the bench in a well lit laboratory (ordinary room lighting). The samples were analyzed after 19 hours of light exposure or more.

The samples were analyzed by HPLC using aHPLC_KB_001, with the HPLC conditions detailed below.

| | |
|---|---|
| HPLC-DAD high pressure system: | Agilent, LC system 1100 series |
| Analytical column: | Supelcosil C-18, 4.6 x 250 mm, 5 µm |
| Column temperature: | : 40 °C |
| Flow rate: | 1 mL/min |
| Injection volume: | 8 µL |
| UV-detection: | 270 nm/340 nm |
| Run time: | 20 min |
| Eluent system: | 50% Solvent C: MeOH/Water with 0.1% v/v acetic acid |
| (95/5 v/v) | 50% Solvent D: Isopropanol |

An overview of the results for the material tested is given in Table 1 and 2. The content of MK-7 and the MK-7 derivative in the samples have been quantified as % area of the total peak area in the chromatogram. The results shows that in MCT oil, MK-7 is sensitive to light in solution, as approximately 70 % degradation is observed after 24 hours of light exposure. For the analogues of MK-7 the result is nearly unchanged after the 24 hours testing period, thus this compound is far more stable towards light exposure. In ethyl acetate MK-7 degrades even more rapidly.

**Table 1: In Ethyl acetate**

| Material | Time point | |
|---|---|---|
| | Initial % | After 19 hrs % |
| MK-7 | 99.4 | 53.7 |
| Example 16 | 93.1 | 92.7 |
| Example 1 | 98.5 | 95.4 |

The results shows that MK-7 is sensitive to light in solution, as approximately 50 % degradation is observed after 19 hours of light exposure.

**Table 2. - in MCT oil**

| Compound | Start purity from CoA (%) | Purity after 1 h (%) | Purity after 1 d (%) | Purity after 3 d (%) |
|---|---|---|---|---|
| MK7 | 99 | 97.2 | 73.5 | 32.2 |
| Example 1 | 98.5 | 98.3 | 97.3 | 92.9 |
| Example 7 | 98.5 | 98.0 | 96.7 | 93.8 |
| Example 13 | 90.2 | 83.6 | 84.8 | 79.6 |

### Example 17 - In vivo testing

### Brief Procedure: Mice

Male mice C57B16 with weights ranging from 38-45 grams were randomised and allocated to different groups with MK-7 compounds or derivatives. Groups had four mice in each group (except ex 2b when only 3 mice were tested). Prior to the experiment mice were allowed to eat regular chow ad libitum. On the day of the experiment, mice were administered MK-7 compounds and derivatives dissolved in ethanol, by oral gavage (2 mg/kg MK-7 equimolar; 100 ul/40 g mouse or MK-7 in corn oil 1mg/kg). At the time of oral gavage, feed but not water was removed.

In venous blood was collected at four hours (300-500 ul) after oral feeding, and then mice were euthanized by cervical dislocation. Blood was collected in tubes coated with EDTA and immediately placed on ice prior to preparation of plasma.

Plasma was prepared by centrifugation at 10,000 g for 10 min, aliquoted and frozen to -20°C until quantification of MK-7.

### Brief Procedure: Rats:

Male Rats aged 6 to 8 weeks and weighing around 180 to 225 g. Animals were fasted overnight with free access to water. Animals were administered test substance by oral gavage with a dose of 100 µg/kg body weight (in recommended formulation and dose volume). Blood samples (150-200 µl) were collected at various time points during the next 48 hours post dose.

To determine the bioavailability of different formulations containing MK-7 in male e Sprague Dawley Rats through gavage. Formulations were both dissolved in sunflower oil. Approximately 0,4ml/animal (depending on the weight of the animal) via oral gavage (100 µg/kg body weight). There were 6 animals per formulation. Blood samples were collected from the tail vein of each animal and transferred into lithium heparin tubes at 2 and 12 hours post dose. Quantification of analyte in plasma was determined by LC-MS-MS analysis: Analyte: MK-7 in plasma. The data are mean of four measurements (low and high values are not included).

**Table 4 - RAT study 0,1mg/kg in oil (sunflower oil)**

| Compound | 2hrs, ng/ml MK-7 | 12hrs ng/ml MK-7 |
|---|---|---|
| MK-7 | 3.6 | 2.7 |
| Example 7 | 2.2 | 2.7 |

The serum level is the same in rats 12 hours after administration for both Ex 7 and MK-7. It is clear that the provitamins give MK-7 in plasma.

### Example 18 - Calcium salt composition

We combined 47 mg of a 0.19 wt% formulation of the MK-7 derivative of Example 16 (diacetate) in microcrystalline cellulose MCC with 645 mg of 90 wt% calcium carbonate, 8 g of magnesium stearate and 745 mg of MCC and formulated tablets of this blend using direct compression.

After formulation we recovered the MK-7 derivative from the tablet. We recovered 88 wt% of the derivative from the tablet.

The same experiment was repeated using identical conditions other than the use of MK-7 rather than the MK-7 derivative. We recovered 77.6 wt% of MK7 from the tablet compared to the amount pre-formualtion. We show therefore that the loss of MK7 during tableting process is 23%.

It has also been found that MK7 degrades in the formulation process together with calcium. This indicates that the MK-7 derivative is more compatible with calcium than MK7.

## Claims

1. A composition, such as a unit dosage form, comprising:
(A) 0.0001 to 10 wt% of a compound of formula (V): 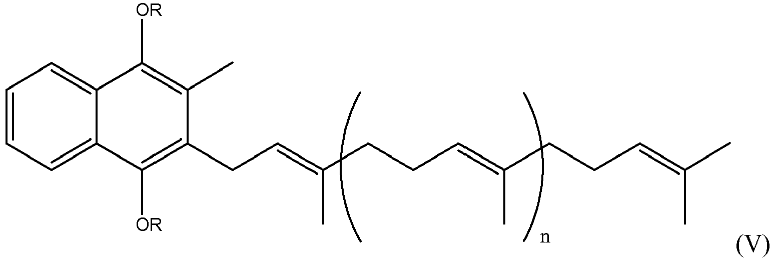 or 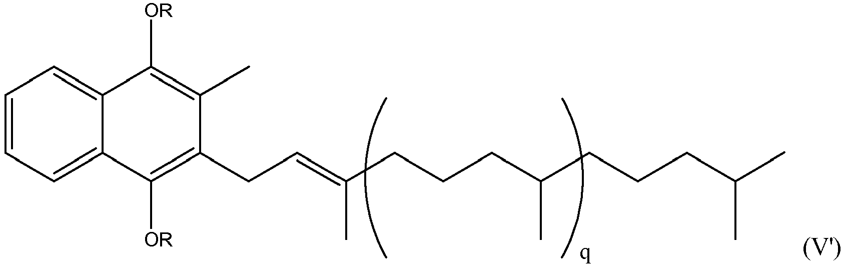
wherein both R groups are -COCH₂Ar, -COAr, or -COC₁₋₆ alkyl group; each Ar is an optionally substituted phenyl or naphthyl group, said substituent being a C1-6 alkyl, CHalH₂, CHal₂H, CHal₃, (where Hal is halide), OH, OC₁₋₆-alkyl, COOR⁶;
each R⁶ is H or C1-6 alkyl;
q is 2;
and n is 4 to 7; or a salt or solvate thereof; and
(B) 10 wt% or more, such as 20 wt% or more of at least one Mg orCa salt.

2. A unit dosage form comprising:
(A) 10 to 500 microg of the compound of formula (V) or (V') as defined in claim 1; and
(B) 10 wt% or more, such as 20 wt% or more of at least one Mg or Ca salt.

3. A composition as claimed in any preceding claim wherein the composition comprises 25 to 200 microg of the compound of formula (V) or (V').

4. A composition as claimed in any preceding claim wherein the composition is a tablet.

5. A composition as claimed in any preceding claim wherein the composition is a tablet formed by direct compression.

6. A composition as claimed in any preceding claim wherein there is at least 40 wt% of calcium salt present in the composition.

7. A composition as claimed in any preceding claim wherein the calcium salt is calcium carbonate.

8. A composition as claimed in any preceding claim wherein both R groups are identical.

9. A composition as claimed in any preceding claim wherein both R groups are COCH₃ or are not COCH₃.

10. A composition as claimed in any preceding claim wherein said compound is or 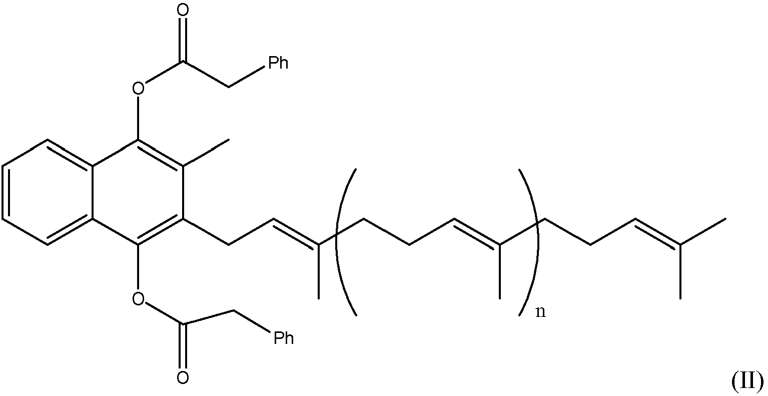 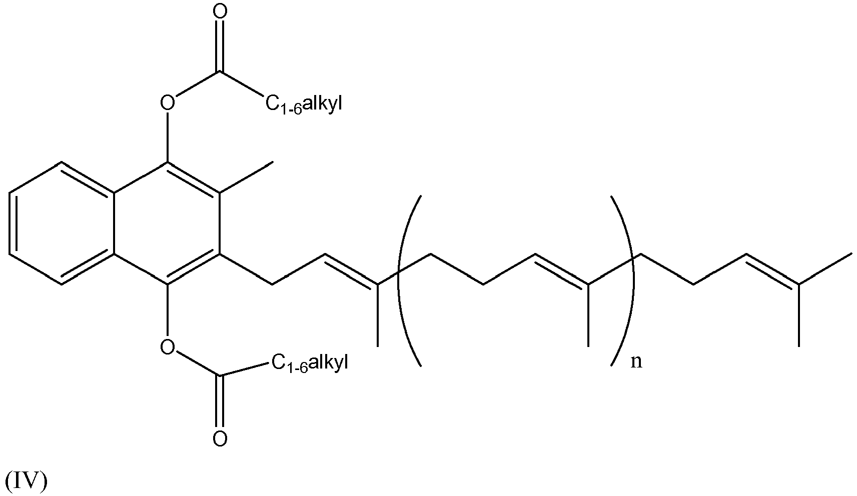 wherein n is 5.

11. A process for the fortification of a food or drink comprising adding to said food or drink a compound of formula (V) or (V') as defined in claim 1; and
at least one Mg orCa salt.

12. A composition as claimed in claim 1 to 10 for use in the treatment of osteoporosis or conditions of the cardiovascular system such as arteriosclerosis.

13. A process for the formation of a unit dosage form comprising blending
(A) 10 to 500 microg of the compound of formula (V) or (V') as hereinbefore defined; and
(B) 10 wt% or more, such as 20 wt% or more of at least one Mg or Ca salt.

## Patentansprüche

1. Zusammensetzung wie in einer Einheitsdosis umfassend:
(A) 0,0001 bis 10 Gew.-% einer Verbindung der Formel (V): 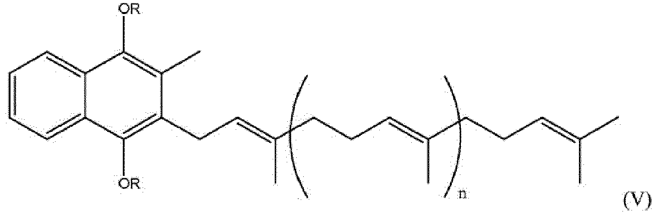 oder 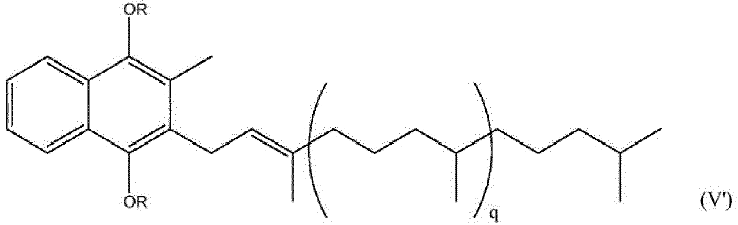
wobei beide R-Gruppen eine -COCH₂Ar-, -COAr- oder -COC₁₋₆-Alkyl-Gruppe sind; jedes Ar eine gegebenenfalls substituierte Phenyl- oder Naphthylgruppe ist, wobei der Substituent ein C1-6-Alkyl, CHalH₂, CHal₂H, CHal₃ (worin Hal Halid ist), OH, OC₁₋₆-Alkyl, COOR⁶ ist;
jedes R⁶ H oder C1-6-Alkyl ist;
q 2 ist;
und n 4 bis 7 ist; oder ein Salz oder Solvat davon; und
(B) 10 Gew.% oder mehr, wie 20 Gew.-% oder mehr wenigstens eines Mg- oder Ca-Salzes.

2. Einheitsdosisform, umfassend
(A) 10 bis 500 microg der Verbindung der Formel (V) oder (V') nach Anspruch 1; und
(B) 10 Gew.-% oder mehr, wie 20 Gew.-% oder mehr wenigstens eines Mg- oder Ca-Salzes.

3. Zusammensetzung nach einem der vorstehenden Ansprüche, wobei die Verbindung 25 bis 200 mircrog der Verbindung der Formel (V) oder (V') enthält.

4. Zusammensetzung nach einem der vorstehenden Ansprüche, wobei die Verbindung eine Tablette ist.

5. Zusammensetzung nach einem der vorstehenden Ansprüche, wobei die Verbindung eine durch direkte Kompresssion geformte Tablette ist.

6. Zusammensetzung nach einem der vorstehenden Ansprüche, wobei wenigstens 40 Gew.-% Calciumsalz in der Zusammensetzung vorliegen.

7. Zusammensetzung nach einem der vorstehenden Ansprüche, wobei das Calciumsalz Calciumcarbonat ist.

8. Zusammensetzung nach einem der vorstehenden Ansprüche, wobei beide R-Gruppen identisch sind.

9. Zusammensetzung nach einem der vorstehenden Ansprüche, wobei beide R-Gruppen COCH₃ oder nicht COCH₃ sind.

10. Zusammensetzung nach einem der vorstehenden Ansprüche, wobei die Verbindung 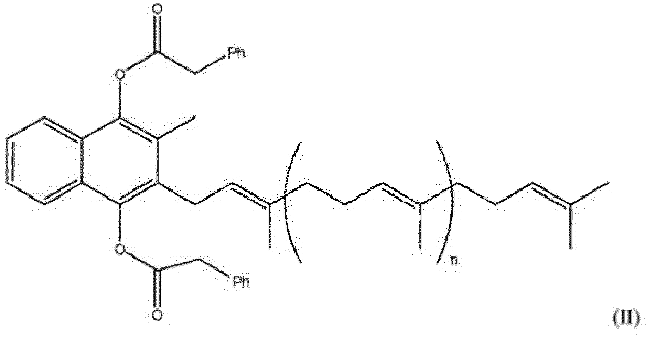 oder 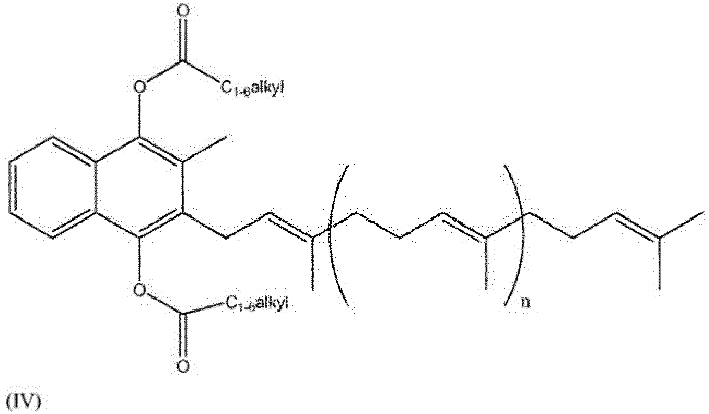 ist, wobei n 5 ist.

11. Verfahren zur Anreicherung eines Lebensmittels oder eines Getränks, umfassend Zugeben zu dem Lebensmittel oder Getränk eine Verbindung der Formel (V) oder (V') nach Anspruch 1; und
wenigstens ein Mg- oder Ca-Salz.

12. Zusammensetzung nach Anspruch 1 bis 10 zur Verwendung in der Behandlung von Osteoporose oder Zuständen des kardiovaskulären Systems, wie Arteriosklerose.

13. Verfahren zur Bildung einer Einheitsdosierungsform umfassend Mischen von
(A) 10 bis 500 microg der Verbindung der Formel (V) oder (V') wie vorstehend definiert und
(B) 10 Gew.-% oder mehr, wie 20 Gew.-% oder mehr wenigstens eines Mg- oder Ca-Salzes.

## Revendications

1. Composition, telle qu'une forme de dosage unitaire, comprenant :
(A) de 0,0001 à 10 % en poids d'un composé de formule (V) : 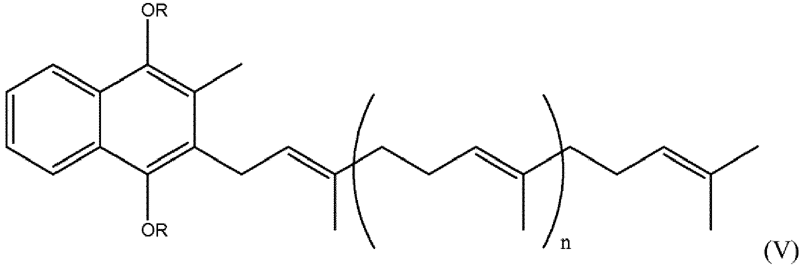 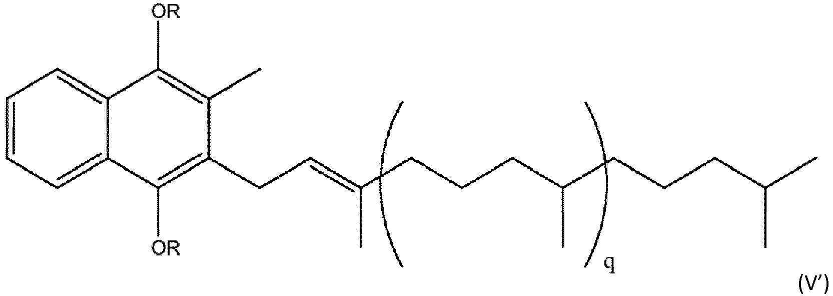 ou
dans laquelle les deux groupes R sont un groupe alkyle -COCH₂Ar, -COAr ou COC₁₋₆ ;
chaque Ar est un groupe phényle ou naphtyle optionnellement substitué, ledit substituant étant un alkyle C1-6, du CHalH₂, du CHal₂H, du CHal₃, (où Hal est un halogénure), du OH, un alkyle OC₁₋₆, du COOR⁶ ;
chaque R⁶ est du H ou un alkyle C1-6 ;
q est 2 ;
et n est de 4 à 7 ; ou un sel ou un solvate de celui-ci ; et
(B) 10 % en poids ou plus, tel que 20 % en poids ou plus d'au moins un sel de Mg ou de Ca.

2. Forme de dosage unitaire comprenant :
(A) de 10 à 500 microgrammes du composé de formule (V) ou (V') tel que défini dans la revendication 1 ; et
(B) 10 % en poids ou plus, tel que 20 % en poids ou plus d'au moins un sel de Mg ou de Ca.

3. Composition selon l'une quelconque des revendications précédentes dans laquelle la composition comprend de 25 à 200 microgrammes du composé de formule (V) ou (V').

4. Composition selon l'une quelconque des revendications précédentes dans laquelle la composition est un comprimé.

5. Composition selon l'une quelconque des revendications précédentes dans laquelle la composition est un comprimé formé par compression directe.

6. Composition selon l'une quelconque des revendications précédentes dans laquelle il y a au moins 40 % en poids de sel de calcium présent dans la composition.

7. Composition selon l'une quelconque des revendications précédentes dans laquelle le sel de calcium est du carbonate de calcium.

8. Composition selon l'une quelconque des revendications précédentes dans laquelle les deux groupes R sont identiques.

9. Composition selon l'une quelconque des revendications précédentes dans laquelle les deux groupes R sont COCH₃ ou ne sont pas COCH₃.

10. Composition selon l'une quelconque des revendications précédentes dans laquelle ledit composé est 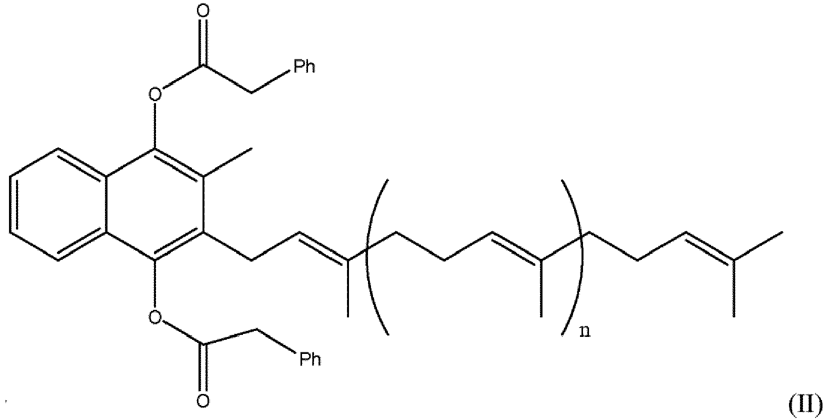 ou 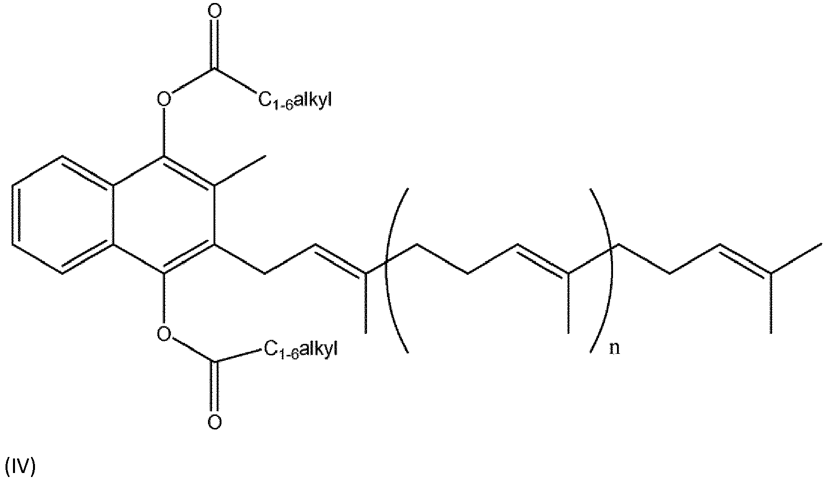 dans laquelle n est 5.

11. Procédé pour la fortification d'un aliment ou d'une boisson comprenant l'ajout audit aliment ou à ladite boisson d'un composé de formule (V) ou (V') tel que défini dans la revendication 1 ; et
au moins un sel de Mg ou de Ca.

12. Composition selon la revendication 1 à 10 à utiliser dans le traitement de l'ostéoporose ou des maladies du système cardiovasculaire comme l'artériosclérose.

13. Procédé pour la formation d'une forme de dosage unitaire comprenant le mélange
(A) de 10 à 500 microgrammes du composé de formule (V) ou (V') tel que défini auparavant ; et
(B) de 10 % en poids ou plus, tel que 20 % en poids ou plus d'au moins un sel de Mg ou de Ca.
